# EUROPEAN PATENT SPECIFICATION

(11) **EP 4 205 682 B1**
(45) Date of publication and mention of the grant of the patent: **15.01.2025**
(21) Application number: 22217138.1
(22) Date of filing: 29.12.2022
(51) Int. Cl.: A61B 18/14

(54) **DUAL BALLOONS FOR PULMONARY VEIN ISOLATION**
DOPPELBALLONS ZUR LUNGENVENENISOLIERUNG
BALLONNETS DOUBLES POUR L'ISOLATION DE VEINE PULMONAIRE

(30) Priority: 30.12.2021 US 202163295344 P; 28.11.2022 US 202218059217
(43) Date of publication of application: 05.07.2023
(73) Proprietor: Biosense Webster (Israel) Ltd., Yokneam, 2066717 (IL)
(72) Inventor: AGNEW, William James, Irvine 92618 (US); MASSOUD, Hamid, Irvine 92618 (US)
(74) Representative: Carpmaels & Ransford LLP

(56) References cited:
- US-A1- 2015 094 701
- US-A1- 2015 157 402
- US-A1- 2020 060 942
- US-A1- 2021 282 847

## Description

### FIELD

The subject matter disclosed herein relates to electrophysiologic catheters, particularly those capable of ablating cardiac tissue.

### BACKGROUND

Ablation of cardiac tissue has been used to treat cardiac arrhythmias. Ablative energies are typically provided to cardiac tissue by a tip portion which can deliver ablative energy alongside the tissue to be ablated. Some of these catheters administer ablative energy from various electrodes located on three-dimensional structures. Ablative procedures incorporating such catheters may be visualized using fluoroscopy.

Ablation catheters with expandable energy-delivery elements are previously known from US 2015/157402 A1, US 2021/282847 A1 and US 2020/060942 A1.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
FIG. 1A is a schematic illustration of an exemplary device for use in a medical procedure;
FIG. 1B illustrates an enlarged portion of inset shown in Fig. 1A;
FIG. 2 illustrates a cross-sectional view of the enlarged portion of Fig. 1B;
FIG 3A illustrates a first example of the devices disclosed herein;
FIG. 3B illustrates a second example of the devices disclosed herein;
FIG. 3C illustrates a third example of the devices disclosed herein;
FIG. 4A illustrates a first mode of operation of the devices disclosed herein;
FIG. 4B illustrates a second mode of operation of the devices disclosed herein;
FIG. 4C illustrates a third mode of operation of the devices disclosed herein;
FIGS. 5A, 5B and 5C illustrate a prototype with a model pulmonary vein; and
FIG. 6 illustrates the overall environment and system used in conjunction with the devices disclosed herein for a cardiac ablation procedure.

### MODES OF CARRYING OUT THE INVENTION

The invention is defined in independent claim 1.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g., "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### Overview

Ablation of cardiac tissue to correct a malfunctioning heart is a well-known procedure for implementing such a correction. Typically, to successfully ablate, cardiac electropotentials need to be measured at various locations of the myocardium. In addition, temperature measurements during ablation provide data enabling the efficacy of the ablation to be measured. Pulmonary vein isolation (PVI) is a technique using a balloon to form a circumferential lesion in the pulmonary veins via a balloon end effector which applies heat ablation via electrodes mounted on the outer surface of the balloon or by cryogenic cooling fluid pumped inside the balloon. With cryogenic cooling, the very low temperature of the cryofluid causes the balloon outer surface to adhere strongly to the cardiac tissues (i.e., the sticky icicle effect) such that a seal is formed between the balloon surface and the tissues. With electrical ablation, there is no icicle effect and hence the operator must apply constant pressure to the balloon to ensure good contact despite the pumping of blood through the veins.

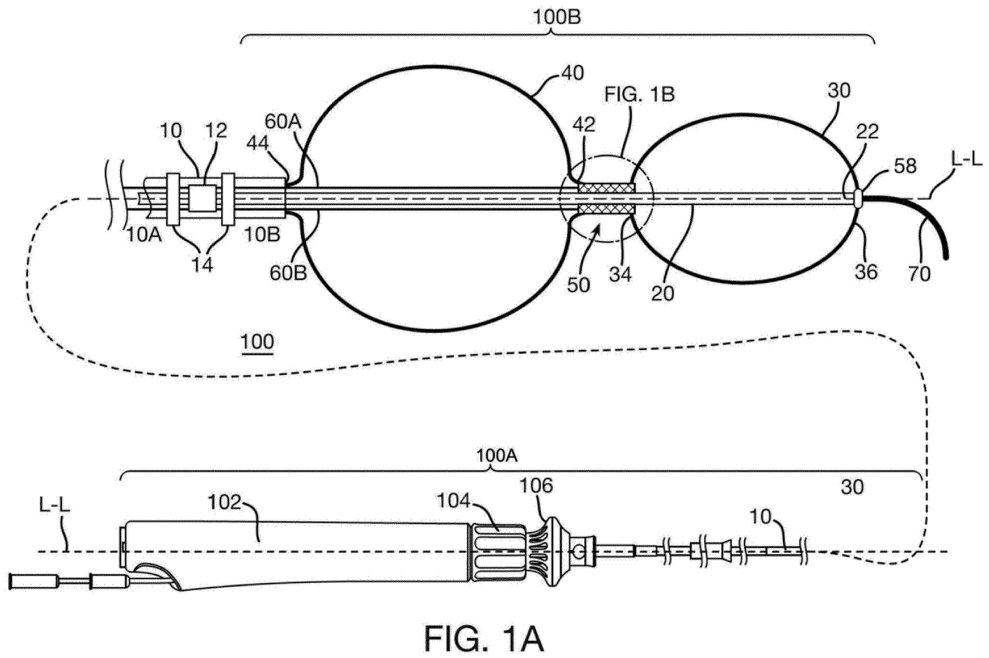

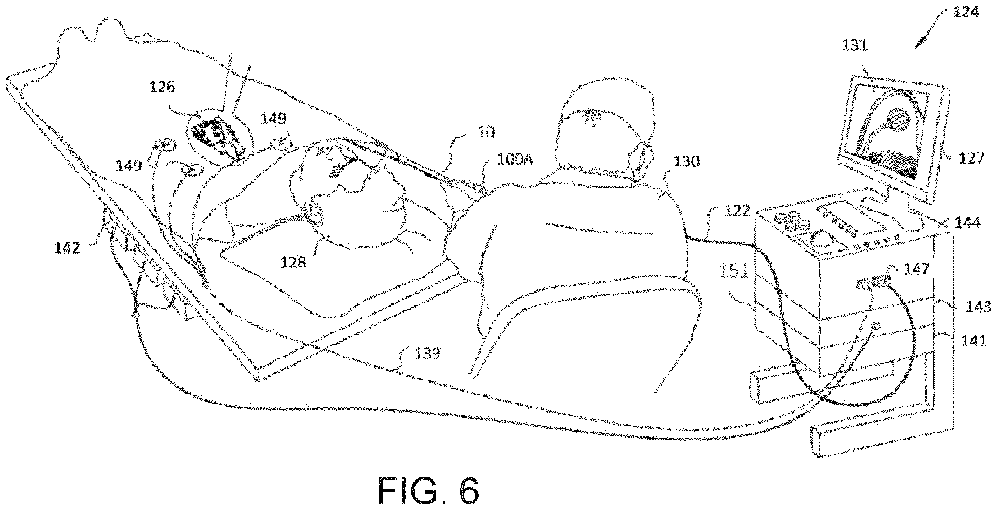

We have devised a balloon type medical catheter 100 that can be used for pulmonary vein isolation via electrical ablation (radiofrequency or irreversible electroporation). As shown in Fig. 1A, catheter 100 is made of two parts extending along a longitudinal axis L-L: a handle assembly 100A and end effector 100B. Handle assembly 100A includes handle 102 provided at a proximal location (proximal being nearest to the operator) with a rotatable wheel 104 that rotates a worm wheel to allow for pinion to translate at least one or more puller wires. The handle 102 also include a pusher or puller member 106 that allows for an actuator shaft 20 disposed inside the catheter shaft 10 to be translated along the longitudinal axis L-L. End effector 100B includes the catheter shaft in the form of a tubular member 10 extending from the proximal handle 100A or a proximal portion 10A to a distal portion 10B along a longitudinal axis L-L. Mounted in the tubular shaft 10 are magnetic location sensing coils 12 (dual axes or triple axes coils) and mounted on the shaft 10 are impedance electrodes 14 that can be used in conjunction with magnetic sensing coils 12 to provide location information of the electrodes 12 (and therefore the location of tubular shaft 10). Techniques for location sensing with magnetic coils 12 and impedance electrodes 14 are well known in the art and will be discussed in context with reference to Fig. 6.

Disposed partially inside the tubular member 10 is an actuator shaft 20 designed to be movable relative to tubular member 10 along axis L-L via pusher 106. Attached to the distal end 22 of actuator 20 is first balloon 30 connected to the distal end 22 via a first balloon distal portion 32 coupled to the actuator shaft distal end 22. The first balloon 30 is coupled to a slidable coupler 50 disposed proximal to the distal end 22. The first balloon has a first balloon proximal portion 34 coupled to the slidable coupler so that there is no movement between first balloon proximal portion 34 and the coupler 50. A second balloon 40 is provided proximal to the coupler 50. The second balloon has a second balloon proximal portion 44 coupled to the tubular member 10 and a second balloon distal portion 42 coupled to the slidable coupler 50. In this exemplary embodiment, the actuator shaft may include a through passage to allow for a guidewire 70 to extend through tubular member 10 all the way to the handle 102.

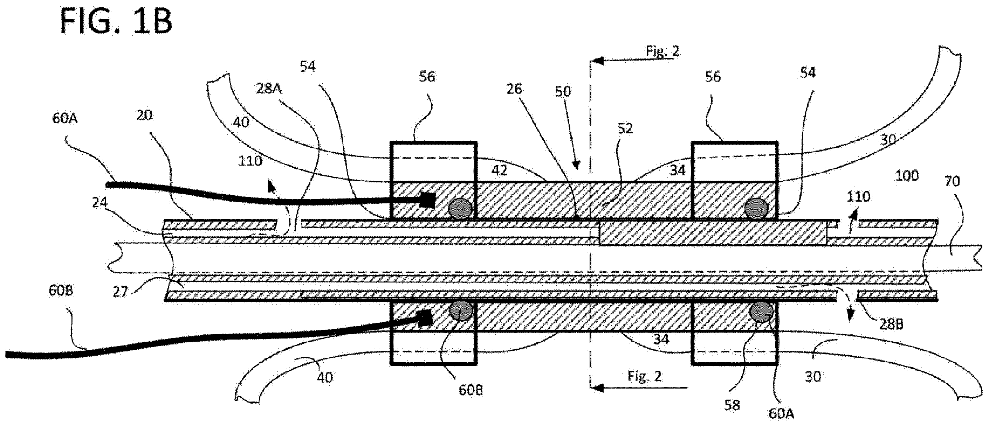

Referring to the inset of Fig. 1B, it can be seen that the coupler 50 is used to connect the first balloon 30 and the second balloon 40 while allowing the coupler 50 to be slidable along axis L-L on actuator shaft 20. It should be noted that coupler 50 has a coupler body 52 with an opening 54 extending along the longitudinal axis through the coupler body 52. The through opening 54 allows for movement of the coupler body 52 relative to the actuator shaft 20 and provide a sealing fit or sliding seal 26 between the coupler body 52 with the actuator shaft 20.

First balloon 30 has its proximal portion 34 captured between a ring retainer 56 and the outer surface of coupler 34. That is, the first balloon 30 is physically connected to the slidable coupler with a coupling ring 56 disposed over a proximal end portion 34 of the first balloon 30 and the coupler body 52 of the slidable coupler 50 so that the proximal end portion of the first balloon is captured between the coupling ring 56 and the coupler body 52. Once the retainer 56 is placed over the balloon distal portion 34, the retainer 56 can be connected to the coupler body 52 via adhesive bonding or mechanical crimping. The distal portion 36 of first balloon can be captured between the actuator shaft and a retainer nosepiece 58 which may have a similar configuration to retainer ring 56 so that nosepiece 58 can be crimped or bonded via adhesive to the actuator shaft. Second balloon 40 has its distal portion 42 captured between ring retainer 56 and the outer surface of coupler 52 and connected to the coupler via adhesive or mechanical retention techniques. The proximal portion 44 of second balloon 40 is captured inside the tubular member 10 via a suitable retention technique e.g., adhesives or polymer reflow). Retainer rings 56 as well as nose piece 58 can be electrically connected (via electrical traces or wires disposed in actuator shaft) to a

The coupler 50 can be coupled to at least one puller wire 60A and configured to be movable relative to shaft 20 such that the coupler 50 is forced to slide along actuator shaft 20. That is, as the at least one puller wire 60A is retracted towards the proximal portion of the tubular member 10, the slidable coupler 50 translates relative to the actuator shaft 20. While two puller wires are shown diametrically disposed for symmetry, only one wire is necessary to allow for translation of the coupler 50. The puller wire 60A or 60B can be insert molded bonded to the coupler 50.

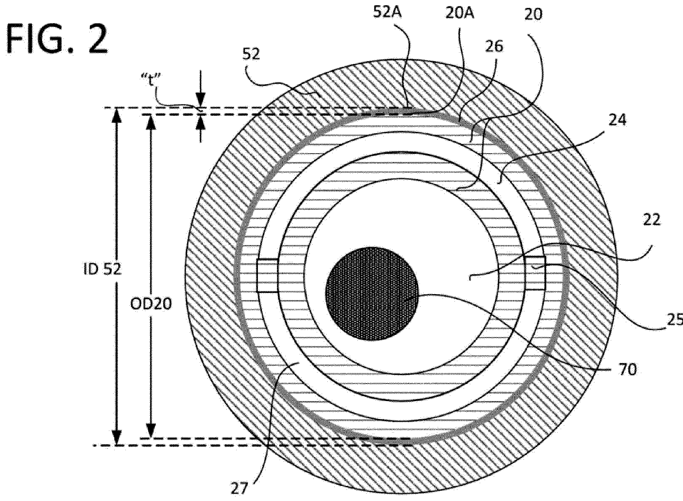

As noted earlier, actuator shaft 20 includes a first passage 22 to allow insertion of a guide wire 70 into the first passage 22 and a second passage 24 generally parallel with the first passage 22 to allow fluid 100 to flow through the second passage 24 and exit through at least one peripheral port 28. The second passage 24 is connected to an irrigation pump with its own valve to control flow of fluid into second passage 24 and out into the second balloon. Also built into the actuator shaft is a third passage 27 to allow fluid flow to the first balloon 30 independently of fluid flow to the second balloon 40. The third passage 27 is also connected to the irrigation pump via its own valves to control flow of fluid into third passage 27 and balloon 30. As shown in Fig. 2, the first and second passages are separated by divider block 25 that extends all the way back to respective valves (not shown) of the second and third passageways. The second and third passages allow for independent inflation of the balloons as will be discussed later with respect to the modes of operation.

With reference to Fig. 2, actuator 20 has an outer diameter OD20 as measured diametrically between its outer surfaces that is slightly less an inside diameter ID52 of inside surface 52A. Disposed between this gap, indicated as "t" is a suitable seal 26 such as a solid type of lubricant coating that ensures a substantial sliding seal between the actuator shaft 20 and the coupler inside surface 52A. In the preferred embodiment, the thickness of "t" for the sliding seal 26 is approximately 5-20 microns in the form of a coating 26 on the on an outer surface 20A of the actuator shaft 20. The thickness t should be a thickness t greater than a difference of an outside diameter of the actuator shaft OD20 and an inside diameter ID52 of the coupler body 52. Alternatively, or in addition thereto, a coating 26 can be disposed on an inside surface 52A of the coupler body 52 having a thickness t greater than a difference of an outside diameter of the actuator shaft and an inside diameter ID52 of the coupler body 52. Alternatively, the coating 26 can be eliminated and instead, the actuator shaft can be made of a lubricious material e.g., Teflon with the outer diameter OD20 being substantially the same or slightly larger than the inside diameter ID52 of coupler body 52 so that a sufficiently tight seal can be formed between the actuator shaft 20 and the coupler body 52 while still allowing for movement of coupler 52 relative to actuator shaft 20. In yet another variation where the outside diameter OD20 of actuator shaft is the same or less than the inside diameter ID52 of coupler body 52, at least one O-ring receiving groove can be formed on the inside surface of the coupler, as shown in Fig. 1B to receive O-ring 60A. A second O-ring groove can also be provided to receive a second O-ring 60B.

The balloon can be assembled as follows. The actuator shaft 20 is inserted into the second balloon 40, coupler 50 (with retainer rings 56 mounted on coupler 50) and first balloon 30 with these components mounted on a jig (not shown) to ensure the correct spacings between the balloons and coupler. Once the correct spacings are made, the proximal portion 44 of second balloon 40 can be bonded to tubular shaft 10. Retainer ring 56 is bonded to the distal portion 42 of second balloon 40 and the coupler 50 while the other retainer ring 56 is bonded to the proximal portion 34 of first balloon 30. The nosepiece can be used to bond the distal portion 36 of first balloon to a distal portion of actuator shaft 20.

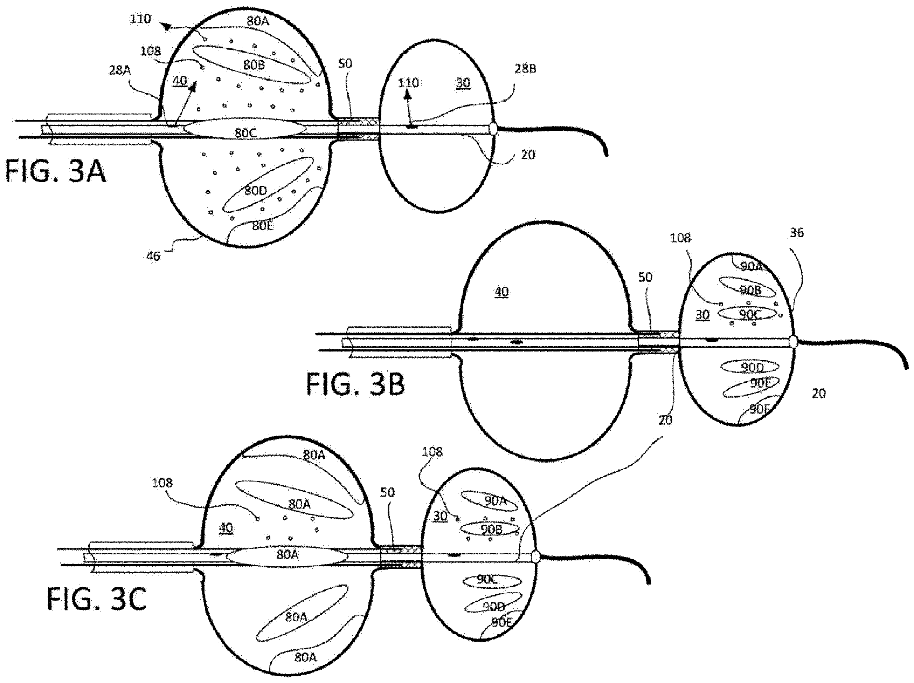

Referring to Fig. 3A, it can be seen that the second balloon 40 may have a plurality of electrodes 80A-80E disposed on an outer surface 46 of the second balloon 40. In an exemplary embodiment, the second balloon may have eight to ten electrodes disposed equiangularly about the longitudinal axis on the outer surface 46 of the second balloon. In yet another embodiment shown in Fig. 3B, the first balloon 30 may include a plurality of electrodes 90A-90F disposed on an outer surface 36 of the first balloon. In Fig. 3C, the first balloon 30 includes a plurality of electrodes 90A-90J disposed on an outer surface 36 of the first balloon and the second balloon 40 also includes a plurality of electrodes 80A-80J disposed on an outer surface 46 of the second balloon 40. It should be noted that pores 108 can be provided through the balloon surface to allow irrigation fluid to escape out of the balloon as a way to irrigate the electrode during ablation. In Figures 3A-3C, each balloon may have eight to twelve electrodes such as the ten-electrode configuration described and illustrated in US Patent No. US 10660700 B2.

In the preferred example of Fig. 3A, the first balloon does not have any electrodes (and therefore no pores 108 are provided) while ablation electrodes are provided on the second balloon 40 with pores for irrigating the electrodes.

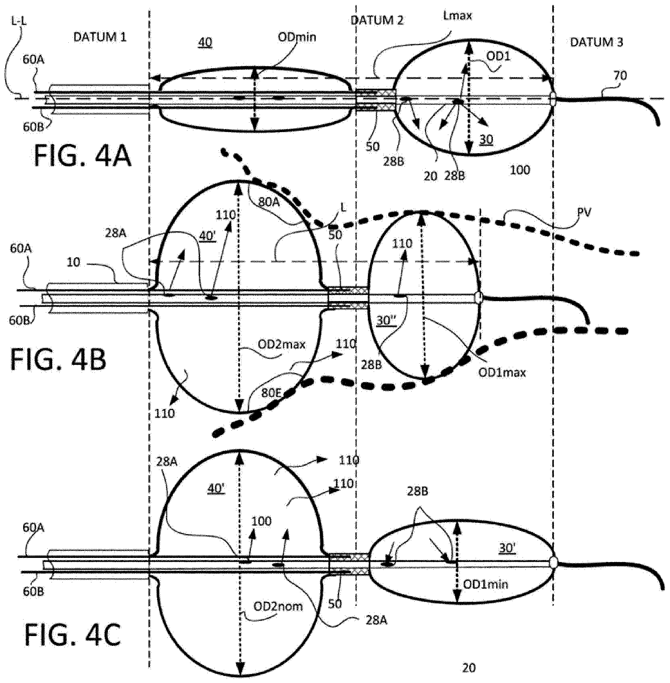

Figures 4A, 4B and 4C illustrate at least three configurations that can be achieved by the invention. In Fig. 4A, the first balloon 30 is shown slightly inflated with the second balloon 40 deflated to illustrate the ability of the inventive concept to utilize independent inflation. In the intended use, the first balloon 30 (slightly inflated with the actuator shaft fully extended to its maximum length Lmax would be inserted into a pulmonary vein to a sufficient length inside the pulmonary vein and inflated slightly (Fig. 4A) to ensure the correct insertion length into the pulmonary vein. The insertion length can be determined via impedance measurement with the magnetic location sensors 12 and via impedance measurement of electrodes 12, nosepiece 58 and the retainer rings 56 so that when the second balloon is inflated all of the electrodes 80 on the second balloon would have good contact with the pulmonary vein before electrical energy is applied. A discussion on magnetic sensors 12 with impedance electrodes 14, 56 and 58 are provided with reference to Fig. 6.

Once the first balloon 30 is positioned at the desired position, the tubular shaft 10 can be remain stationary while the first balloon 30 is fully inflated via port 28B to its maximum diameter. As the first balloon is inflated, the length of the first balloon 30 shortens causing the actuator shaft 20 to retract with respect to the coupler 50. Once fully inflated, the first balloon 30 will remain locked to the walls of the pulmonary vein PV. At this time, the second balloon can be inflated with fluid 110 via port 28A (independent from port 28B. Due to porosity of the balloon, some fluid 110 will escape the interior of second balloon 40. The operator can adjust the size of second balloon 40 by holding tube 10 stationary while retracting shaft 20. Because the first balloon 30 does not have pores, inflation pressure remains constant and therefore its maximum outer diameter ODlmax remains constant, adjustment of the shaft 20 proximally causes the slidable coupler 50 to automatically adjust proximally shortening the longitudinal length of second balloon 40 thereby increasing its diameter to a maximum diameter of OD2max. At this juncture, the operator can apply ablation energy (as illustrated and described in relation to Fig. 6) to the electrodes 80 on the second balloon 40'. Once the ablation is completed, the first balloon 30 can be deflated, shown here in Fig. 4C with fluid being evacuated from the ports 28B. The second balloon 40 can remain inflated so that impedance measurement to assess the effectiveness of the ablation can be performed. Alternatively, the second balloon 40 can also be deflated at the same time as the first balloon 30.

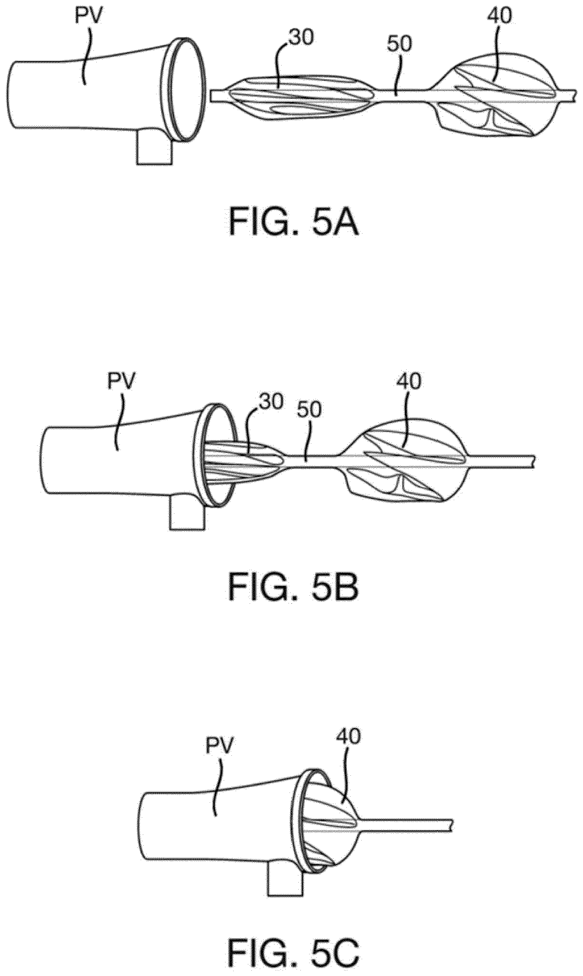

Figs. 5A, 5B and 5C illustrates a prototype in a different sequence of inflation as another technique that can be utilized by one skilled in the art. In Fig. 5A, the first balloon 30 is not inflated to allow for entry into a small model of the pulmonary vein PV in Fig. 5B. The second balloon 40 can be slightly inflated as the first balloon approaches the PV. In Fig.5C, the first balloon 30 is fully inside the PV while the second balloon 40 is now presenting its equator (and therefore ablation electrodes 80 [not shown]) for ablation of the entrance to the pulmonary vein PV. At this stage, the second balloon 40 can be inflated first then the first balloon 30 can be inflated next to ensure securement of the second balloon to the PV. Alternatively, the first balloon 30 can be inflated first and the second balloon 40 can be inflated next and thereafter ablation energy can be delivered to the electrodes as described in Fig. 4C.

Fig. 6 is pictorial illustration of a mapping system 124, in accordance with an exemplary mode of the present disclosure. The system shown here includes a catheter shaft 10 configured to be inserted into a body part (e.g., a chamber of a heart 126) of a living subject (e.g., a patient 128). A physician 130 navigates the end effector 100B described and illustrated herein to a target location in the heart 126 of the patient 128, by manipulating a deflectable segment of the end effector. Patient 128 is placed in a magnetic field generated by a pad containing multiple magnetic field generator coils 142, which are driven by a unit 143. The magnetic field generator coils 142 are configured to generate respective alternating magnetic fields, having respective different frequencies, into a region where a body-part (e.g., the heart 126) of a living subject (e.g., the patient 128) is located. The magnetic coil sensors 12 are configured to output electrical signals responsively to detecting the respective magnetic fields. For example, if there are nine magnetic field generator coils 142 generating nine respective different alternating magnetic fields with nine respective different frequencies, the electrical signals output by the magnetic coil sensors 12 will include components of the nine different frequency alternating magnetic fields. The magnitude of each of the magnetic fields varies with distance from the respective magnetic field generator coils 142 such that the location of the magnetic coil sensors 12 may be determined from the magnetic fields sensed by the magnetic coil sensors 12. Therefore, the transmitted alternating magnetic fields generate the electrical signals in sensors 12, so that the electrical signals are indicative of position and orientation of the magnetic coil sensors 12. It is noted that the magnetic coil sensors can be triple-axis-sensor to measure three different axes of orientation.

In some exemplary modes, the processing circuitry 141 uses position-signals received from the electrodes 14 or body surface electrodes 149, and the magnetic sensor 12 to estimate a position of the assembly 100B inside a body part, such as inside a cardiac chamber. In some exemplary modes, the processing circuitry 141 correlates the position signals received from the electrodes 14 and 149 with previously acquired magnetic location-calibrated position signals, to estimate the position of the assembly 100B inside the body part. The position coordinates of the electrodes 14 may be determined by the processing circuitry 141 based on, among other inputs, measured impedances, voltages or on proportions of currents distribution, between the electrodes 14 and the body surface electrodes 149.

The method of position sensing using current distribution measurements and/or external magnetic fields is implemented in various medical applications, for example, in the Carto^{®} system, produced by Biosense Webster Inc. (Irvine, California), and is described in detail in U.S. Patents 5,391,199, 6,690,963, 6,144,118, 6,239,724, 6,618,612, 6,332,089, 7,756,576, 7,869,865, and 7,848,787, in PCT Patent Publication WO 96/05768, and in U.S. Patent Application Publications shown here US2002/0065455 A1 and shown here US2004/0068178 A1.

The Carto^{®}3 system applies Active Current Location (ACL) which is a hybrid current-distribution and magnetic-based position-tracking technology. In some exemplary modes, using ACL, the processing circuitry 141 estimates the positions of the electrodes 14. In some exemplary modes, the signals received from the electrodes 14, 149 are correlated with a current-to-position matrix (CPM) which maps current distribution ratios (or another electrical value) with a position that was previously acquired from magnetic location-calibrated position signals. The current distribution ratios are based on measurements of the body surface electrodes 149 of current flowing from the electrodes 14 to the body surface electrodes 149.

In some exemplary modes, to visualize catheters which do not include a magnetic sensor, the processing circuitry 141 may apply an electrical signal-based method, referred to as Independent Current Location (ICL) technology. In ICL, the processing circuitry 141 calculates a local scaling factor for each voxel of the volume in which catheters are visualized. The factor is determined using a catheter with multiple electrodes having a known spatial relationship, such as a lasso-shaped catheter. However, although yielding accurate local scaling (e.g., over several millimeters), ICL is less accurate when applied to a volume of a whole heart chamber, whose size is in the order of centimeters. The ICL method, in which positions are calculated based on current distribution proportions can have errors and may yield a distorted shape of the assembly 100B, due to the non-linear nature of the current-based ICL space. In some exemplary modes, the processing circuitry 141 may apply the disclosed ICL method to scale ICL space and the assembly 100B shape into a correct one, based on known smaller scale distances between electrodes 80A-80J.

Processing circuitry 141, typically part of a general-purpose computer, is further connected via a suitable front end and interface circuits 44, to receive signals from body surface-electrodes 149. Processing circuitry 141 is connected to surface-electrodes 149 by wires running through a cable 139 to the chest of patient 128. The catheter 100A includes a connector 147 disposed at the proximal end 100A of the insertion tube 10 for coupling to the processing circuitry 141.

In some exemplary modes, processing circuitry 141 renders to a display 127, a representation 131 of at least a part of the catheter 100a and an anatomical map or body-part, (e.g., from a mapping process or from a scan (e.g., CT or MRI) of the body-part previously registered with the system shown here), responsively to computed position coordinates of the insertion tube 10 and the electrodes 80A-80J on the ablation balloon.

Processing circuitry 141 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory. The system shown here may also include a memory 151 used by the processing circuitry 141.By virtue of the components and system described and illustrated herein, a method of operating a balloon type ablation effector can be achieved, however, this method itself is not claimed. The non-claimed method can be achieved with an end effector having a tubular member 10 extending from a proximal portion 10A to a distal portion 10B along a longitudinal axis L-L with an actuator shaft 20 extending from inside the tubular member 10 to an actuator shaft distal end 22, the actuator shaft having a plurality of fluid ports. The end effector 100B includes a first balloon 30 comprising a first balloon distal portion 32 coupled to the actuator shaft distal end 22 and a first balloon proximal portion 34 coupled to a slidable coupler 50. The end effector 100 also includes a second balloon 40 comprising a second balloon proximal portion 44 coupled to the tubular member 10 and a second balloon distal portion 42 coupled to the slidable coupler 50. The steps in the non-claimed method include extending the actuator shaft in a distal direction to a maximum length of the actuator shaft relative to the tubular member; inflating at least one of the first and second balloons with fluid via fluid ports so the first balloon reaches a first balloon outer diameter and the second balloon reaches a second balloon diameter increasing the second balloon outer diameter of the second balloon to a maximum outer diameter and decreasing the first balloon outer diameter of the first balloon by retraction of the coupler body relative to the tubular shaft.

Other steps include increasing the first balloon outer diameter to a maximum outer diameter by retraction of the actuator shaft along the longitudinal axis towards the proximal portion of the tubular shaft.

The teachings, expressions, embodiments, examples, etc. described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the systems described herein may be accomplished within the scope of the invention as defined in claim 1 by appropriate modifications.

## Claims

1. A balloon type medical apparatus (100) comprising:
a tubular member (10) extending from a proximal portion to a distal portion along a longitudinal axis;
an actuator shaft (20) extending from inside the tubular member to an actuator shaft distal end;
a first balloon (30) comprising a first balloon distal portion coupled to the actuator shaft distal end and a first balloon proximal portion coupled to a slidable coupler (50);
a second balloon (40) comprising a second balloon proximal portion coupled to the tubular member and a second balloon distal portion coupled to the slidable coupler (50), in which the slidable coupler includes:
a coupler body (52) with an opening (54) extending along the longitudinal axis through the coupler body, the opening configured to allow movement of the coupler body relative to the actuator shaft and provide a sealing fit between the coupler body with the actuator shaft.

2. The apparatus of claim 1, in which the first balloon is connected to the actuator shaft distal end with a nose piece disposed over a distal end portion of the first balloon to capture the distal end portion of the balloon between the nose piece and the actuator shaft.

3. The apparatus of claim 1, in which the first balloon is connected to the slidable coupler with a coupling ring disposed over a proximal end portion of the first balloon and the coupler body of the slidable coupler so that the proximal end portion of the first balloon is captured between the coupling ring and the coupler body.

4. The apparatus of claim 1, in which a distal end portion of the second balloon is captured between a coupling ring and the coupler body.

5. The apparatus of claim 1, in which at least one puller wire is connected to the coupler body so that when the at least one puller wire is retracted towards the proximal portion of the tubular member, the slidable coupler translates relative to the actuator shaft.

6. The apparatus of claim 5, in which the at least one puller wire comprises two puller wires disposed diametrically with respect to the longitudinal axis.

7. The apparatus of claim 1, in which that actuator shaft includes a first passage to allow insertion of a guide wire into the first passage and a second passage generally parallel with the first passage to allow fluid to flow through the second passage and exit through at least one peripheral port.

8. The apparatus of claim 1, in which the actuator shaft includes a first passage to allow insertion of a guidewire, a second passage to provide fluid flow to enter the second balloon via a fluid port and a third passage to allow fluid flow to the first balloon.

9. The apparatus of claim 1, in which the sealing fit comprises a solid lubricant coating disposed on an outer surface of the actuator shaft having a thickness greater than a difference of an outside diameter of the actuator shaft and an inside diameter of the coupler body.

10. The apparatus of claim 1, in which the sealing fit comprises a solid lubricant coating disposed on an inside surface of the coupler body having a thickness greater than a difference of an outside diameter of the actuator shaft and an inside diameter of the coupler body.

11. The apparatus of claim 1, in which the second balloon includes a plurality of electrodes disposed on an outer surface of the second balloon.

12. The apparatus of claim 1 or claim 11, in which the first balloon includes a plurality of electrodes disposed on an outer surface of the first balloon.

13. The apparatus of claim 1, in which the actuator shaft includes a primary irrigation passage to deliver fluid to the second balloon.

14. The apparatus of claim 1, in which the actuator shaft includes a secondary irrigation passage to deliver fluid to the first balloon independent of delivery of fluid to the second balloon.

## Patentansprüche

1. Ballonartige medizinische Vorrichtung (100), umfassend:
ein rohrförmiges Element (10), das sich von einem proximalen Abschnitt zu einem distalen Abschnitt entlang einer Längsachse erstreckt;
eine Betätigungswelle (20), die sich von einem Inneren des rohrförmigen Elements zu einem distalen Ende der Betätigungswelle erstreckt;
einen ersten Ballon (30), umfassend einen ersten distalen Ballonabschnitt, der mit dem distalen Ende der Betätigungswelle gekoppelt ist, und einen ersten proximalen Ballonabschnitt, der mit einem gleitfähigen Koppler (50) gekoppelt ist;
einen zweiten Ballon (40), umfassend einen zweiten proximalen Ballonabschnitt, der mit dem rohrförmigen Element gekoppelt ist, und einen zweiten distalen Ballonabschnitt, der mit dem gleitfähigen Koppler (50) gekoppelt ist, wobei der gleitfähige Koppler einschließt:
einen Kopplerkörper (52) mit einer Öffnung (54), die sich entlang der Längsachse durch den Kopplerkörper erstreckt, wobei die Öffnung konfiguriert ist, um eine Bewegung des Kopplerkörpers relativ zu der Betätigungswelle zu ermöglichen und eine Dichtungspassung zwischen dem Kopplerkörper mit der Betätigungswelle bereitzustellen.

2. Vorrichtung nach Anspruch 1, wobei der erste Ballon mit dem distalen Ende der Betätigungswelle verbunden ist, wobei ein Nasenstück über einem distalen Endabschnitt des ersten Ballons angeordnet ist, um den distalen Endabschnitt des Ballons zwischen dem Nasenstück und der Betätigungswelle zu erfassen.

3. Vorrichtung nach Anspruch 1, wobei der erste Ballon mit dem gleitfähigen Koppler über einen Kopplungsring verbunden ist, der über einem proximalen Endabschnitt des ersten Ballons und dem Kopplerkörper des gleitfähigen Kopplers angeordnet ist, so dass der proximale Endabschnitt des ersten Ballons zwischen dem Kopplungsring und dem Kopplerkörper erfasst ist.

4. Vorrichtung nach Anspruch 1, wobei ein distaler Endabschnitt des zweiten Ballons zwischen einem Kopplungsring und dem Kopplerkörper erfasst ist.

5. Vorrichtung nach Anspruch 1, wobei der mindestens eine Zugdraht mit dem Kopplerkörper verbunden ist, so dass sich der gleitfähige Koppler relativ zu der Betätigungswelle verschiebt, wenn der mindestens eine Zugdraht zu dem proximalen Abschnitt des röhrenförmigen Elements hin zurückgezogen wird.

6. Vorrichtung nach Anspruch 5, wobei der mindestens eine Zugdraht zwei Zugdrähte umfasst, die diametral zu der Längsachse angeordnet sind.

7. Vorrichtung nach Anspruch 1, wobei die Betätigungswelle einen ersten Durchgang, um ein Einführen eines Führungsdrahtes in den ersten Durchgang zu ermöglichen, und einen zweiten Durchgang einschließt, der im Allgemeinen parallel zu dem ersten Durchgang verläuft, um zu ermöglichen, dass Flüssigkeit durch den zweiten Durchgang strömt und durch mindestens einen peripheren Anschluss austritt.

8. Vorrichtung nach Anspruch 1, wobei die Betätigungswelle einen ersten Durchgang, um das Einführen eines Führungsdrahtes zu ermöglichen, einen zweiten Durchgang, um einen Flüssigkeitsstrom bereitzustellen, um über einen Flüssigkeitsanschluss in den zweiten Ballon einzutreten, und einen dritten Durchgang, um den Flüssigkeitsstrom zu dem ersten Ballon zu ermöglichen, einschließt.

9. Vorrichtung nach Anspruch 1, wobei die Dichtungspassung eine Beschichtung aus festem Schmiermittel umfasst, die auf einer Außenoberfläche der Betätigungswelle angeordnet ist, die eine Dicke aufweist, die größer ist als eine Differenz zwischen einem Außendurchmesser der Betätigungswelle und einem Innendurchmesser des Kopplerkörpers.

10. Vorrichtung nach Anspruch 1, wobei die Dichtungspassung eine Beschichtung aus festem Schmiermittel umfasst, die auf einer Innenoberfläche der Betätigungswelle angeordnet ist, die eine Dicke aufweist, die größer ist als eine Differenz zwischen einem Außendurchmesser der Betätigungswelle und einem Innendurchmesser des Kopplerkörpers.

11. Vorrichtung nach Anspruch 1, wobei der zweite Ballon eine Vielzahl von Elektroden einschließt, die auf einer Außenoberfläche des zweiten Ballons angeordnet sind.

12. Vorrichtung nach Anspruch 1 oder 11, wobei der erste Ballon eine Vielzahl von Elektroden einschließt, die auf einer Außenoberfläche des ersten Ballons angeordnet sind.

13. Vorrichtung nach Anspruch 1, wobei die Betätigungswelle einen primären Spüldurchgang einschließt, um Flüssigkeit an den zweiten Ballon abzugeben.

14. Vorrichtung nach Anspruch 1, wobei die Betätigungswelle einen sekundären Spüldurchgang einschließt, um Flüssigkeit unabhängig von der Abgabe von Flüssigkeit an den zweiten Ballon an den ersten Ballon abzugeben.

## Revendications

1. Appareil médical de type ballon (100), comprenant :
un élément tubulaire (10) s'étendant depuis une partie proximale jusqu'à une partie distale le long d'un axe longitudinal ;
un arbre d'actionneur (20) s'étendant depuis l'intérieur de l'élément tubulaire jusqu'à une extrémité distale d'arbre d'actionneur ;
un premier ballon (30) comprenant une première partie distale de ballon accouplée à l'extrémité distale d'arbre d'actionneur et une première partie proximale de ballon accouplée à un accoupleur coulissant (50) ;
un second ballon (40) comprenant une seconde partie proximale de ballon accouplée à l'élément tubulaire et une seconde partie distale de ballon accouplée à l'accoupleur coulissant (50), dans lequel l'accoupleur coulissant comporte :
un corps d'accoupleur (52) avec une ouverture (54) s'étendant le long de l'axe longitudinal à travers le corps d'accoupleur, l'ouverture conçue pour permettre un mouvement du corps d'accoupleur par rapport à l'arbre d'actionneur et fournir un ajustement étanche entre le corps d'accoupleur et l'arbre d'actionneur.

2. Appareil selon la revendication 1, dans lequel le premier ballon est relié à l'extrémité distale d'arbre d'actionneur avec une pièce de nez disposée sur une partie d'extrémité distale du premier ballon pour capturer la partie d'extrémité distale du ballon entre la pièce de nez et l'arbre d'actionneur.

3. Appareil selon la revendication 1, dans lequel le premier ballon est relié à l'accoupleur coulissant avec un anneau d'accouplement disposé sur une partie d'extrémité proximale du premier ballon et le corps d'accoupleur de l'accoupleur coulissant de sorte que la partie d'extrémité proximale du premier ballon est capturée entre l'anneau d'accouplement et le corps d'accoupleur.

4. Appareil selon la revendication 1, dans lequel une partie d'extrémité distale du second ballon est capturée entre un anneau d'accouplement et le corps d'accoupleur.

5. Appareil selon la revendication 1, dans lequel au moins un fil de traction est relié au corps d'accoupleur de sorte que lorsque l'au moins un fil de traction est rétracté vers la partie proximale de l'élément tubulaire, l'accoupleur coulissant se translate par rapport à l'arbre d'actionneur.

6. Appareil selon la revendication 5, dans lequel l'au moins un fil de traction comprend deux fils de traction disposés diamétralement par rapport à l'axe longitudinal.

7. Appareil selon la revendication 1, dans lequel l'arbre d'actionneur comporte un premier passage pour permettre une insertion d'un fil-guide dans le premier passage et un second passage généralement parallèle au premier passage pour permettre à un fluide de s'écouler à travers le second passage et de sortir par au moins un orifice périphérique.

8. Appareil selon la revendication 1, dans lequel l'arbre d'actionneur comporte un premier passage pour permettre une insertion d'un fil-guide, un deuxième passage pour fournir un flux de fluide pour entrer dans le second ballon par l'intermédiaire d'un orifice de fluide et un troisième passage pour permettre un flux de fluide vers le premier ballon.

9. Appareil selon la revendication 1, dans lequel l'ajustement d'étanchéité comprend un revêtement lubrifiant solide disposé sur une surface externe de l'arbre d'actionneur ayant une épaisseur supérieure à une différence entre un diamètre extérieur de l'arbre d'actionneur et un diamètre intérieur du corps d'accoupleur.

10. Appareil selon la revendication 1, dans lequel l'ajustement d'étanchéité comprend un revêtement lubrifiant solide disposé sur une surface intérieure du corps d'accoupleur ayant une épaisseur supérieure à une différence entre un diamètre extérieur de l'arbre d'actionneur et un diamètre intérieur du corps d'accoupleur.

11. Appareil selon la revendication 1, dans lequel le second ballon comporte une pluralité d'électrodes disposées sur une surface externe du second ballon.

12. Appareil selon la revendication 1 ou la revendication 11, dans lequel le premier ballon comporte une pluralité d'électrodes disposées sur une surface externe du premier ballon.

13. Appareil selon la revendication 1, dans lequel l'arbre d'actionneur comporte un passage d'irrigation primaire pour acheminer un fluide au second ballon.

14. Appareil selon la revendication 1, dans lequel l'arbre d'actionneur comporte un passage d'irrigation secondaire pour acheminer un fluide au premier ballon indépendamment de l'acheminement de fluide au second ballon.
